(19) Europäisches Patentamt
European Patent Office
Office européen des brevets

(11) Publication number: **0 454 867 B1**

# (12) EUROPEAN PATENT SPECIFICATION

(45) Date of publication of patent specification: **01.02.95**

(51) Int. Cl.$^6$: **C07C 45/72**, C07C 49/203, C07C 49/21, C07C 49/217, C07D 335/02, C07D 309/06, C07D 307/46

(21) Application number: **90916813.0**

(22) Date of filing: **15.11.90**

(86) International application number: **PCT/JP90/01487**

(87) International publication number: **WO 91/07370 (30.05.91 91/12)**

## (54) A METHOD FOR THE SYNTHESIS OF -g(a), -g(b)-UNSATURATED KETONES.

(30) Priority: **17.11.89 JP 297387/89**

(43) Date of publication of application: **06.11.91 Bulletin 91/45**

(45) Publication of the grant of the patent: **01.02.95 Bulletin 95/05**

(84) Designated Contracting States: **AT BE CH DE DK ES FR GB GR IT LI LU NL SE**

(56) References cited:
**WO-A-86/02065**
**FR-A- 2 481 701**

**CHEMICAL ABSTRACTS, vol. 105, 1986, page 647, column 2, abstract no. 152541b, Columbus, Ohio, US; T. TSUMOTU et al, "alpha,beta-Unsaturated ketones"; SU-A-1176827**

(73) Proprietor: **NIPPON SODA CO., LTD.**
**2-1, Ohtemachi 2-chome**
**Chiyoda-ku,**
**Tokyo 100 (JP)**

(72) Inventor: **TSUKASHIMA, Keiichi Takaoka Plant**
**Nippon Soda Co., Ltd.**
**300, Mukainohonmachi**
**Takaoka-shi**
**Toyama 933 (JP)**
Inventor: **NAKAJIMA, Masashi Takaoka Plant**
**Nippon Soda Co., Ltd.**
**300, Mukainohonmachi**
**Takaoka-shi**
**Toyama 933 (JP)**
Inventor: **FUJIMARU, Masayoshi Takaoka Plant**
**Nippon Soda Co., Ltd.**
**300, Mukainohonmachi**
**Takaoka-shi**
**Toyama 933 (JP)**
Inventor: **SUZUKI, Kenji Takaoka Plant**
**Nippon Soda Co., Ltd.**
**300, Mukainohonmachi**
**Takaoka-shi**
**Toyama 933 (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

(74) Representative: **de Bruijn, Leendert C. et al**
**Nederlandsch Octrooibureau**
**Scheveningseweg 82**
**P.O. Box 29720**
**NL-2502 LS Den Haag (NL)**

## Description

This invention relates to a process for the preparation of an α,β-unsaturated ketone by reacting an aldehyde with an alkaline metal salt of acetoacetic acid represented by the formula

$$CH_3 - \underset{\underset{O}{\|}}{C}CH_2COO^-M^+$$

wherein $M^+$ is an alkaline metal ion, in the presence of a secondary amine, in a mixed solvent of water and a water-insoluble organic solvent, while keeping the pH constant with mineral acid, and by adjusting the amount of water.

α, β-unsaturated ketones are very useful as intermediates for pharmaceuticals and agricultural chemicals.

Background Art:

Various methods for the synthesis of unsaturated ketones using aldehyde as a starting material have been reported so far. These synthetic methods have various problems in industrial applications. For instance, in the aldol condensation of aldehyde with acetone, generally a large amount of byproducts are produced, isolation of the intended product is difficult, yield is low, and a very excessive amount of acetone is required.

In the synthetic method of condensing aldehyde and acetone using piperidineacetic acid as a catalyst [described in such documents as Indian J. Chem. Vol 16B. 970-972 (1978)] , a large qunatity of expensive catalyst is required, and a very excessive amount of acetone is necessary.

In the synthetic method that a Wittig reagent, which is obtained from the synthesis of monochloroacetone or monobromoacetone and triphenylphosphine, with aldehyde [described in such documents as Ber. 108. 2077 (1970)] , the Wittig reagent of the material is expensive, and waste treatment is difficult. In the method that α, β-unsaturated β' -ketoester, which is obatined from the synthesis of aldehyde and tert-butyl acetoacetate, is pyrolyzed using p-toluenesulfonic acid as a catalyst at a high temperature [described in such documents as Acta Chem. Scand., 17, 2216-220 (1963) ] , synthesis yield is low in spite of low-temperature and many-hour synthesis of α, β-unsaturated β'-ketoester.

Synthesis yield is low in spite of many-hour reaction in the synthetic method that aldehyde and actone are reacted (US. 2,108,427).

In the method of Knoevenagel reaction of an alkaline metal salt of acetoacetic acid and aldehyde in the presence of aliphatic amine, which we applied before, (Japanese open patent No. Sho 57-4930 corresponding to FR-A-2481701), α, β-unsaturated ketone is obtained with good yield if the aldehyde has two hydrogen atoms at the α position. If no or one hydrogen atom at the α position, the reaction is extremely slow and yield is low. The aqueous solution of sodium acetoacetate obtained from hydrolysis of methylacetoacetate with sodium hydroxide is around 30% in concentration. When this aqueous solution and 3-ethylthiobutanal are reacted using piperidine as a catalyst and concentrated hydrochloric acid as a pH regulating agent, the intended α, β-unsaturated ketone is obtained with good yield of 90% or more. However, if an aldehyde branching at the α position, for instance 3-tetrahydrothiopyran carbaldehyde, is reacted under the same conditions, the yield is low.

An object of this invention is to provide methods for the synthesis of α, β-unsaturated ketone with no or one hydrogen atom at the α position, with good yield, in the reaction of alkaline metal salt of acetoacetate and aldehyde.

Disclosure of Invention:

The inventors earnestly studied the aforementioned reaction of alkaline metal salt of acetoacetate and aldehyde with the aim of synthesis of α, β-unsaturated ketone with no or one hydrogen atom at the α position, and found as the result that the low yield when such an aldehyde is reacted results from its steric hindrance and that the structure of the catalyst secondary amine and an amount of water in the system are factors to compensate for it. Thus this invention has been completed.

In other words, as a catalyst, the two carbons adjacent to N of secondary amine should be both methylene if the amine is cyclic and one of them be methyl if straight chain, and furthermore the amine be highly hydrophobic. Therefore, a water-soluble amine, such as piperidine, which is effective to aldehydes

having two hydrogen atoms at the α position, is hardly effective to aldehydes with no or one hydrogen atom at the α position.

Diethylamine, dibutylamine and N-ethyl-n-laurylamine have an extremely small catalytic effect because one of the alkyl groups is not methyl.

An amount of water in the system is important and is required to be reduced. Methods to attain it are concentration of aqueous solution of alkaline metal salt of acetoacetic acid, and/or use of acid gas or acid anhydride or concentrated mineral acid with less water content, as an acid to maintain the pH, and/or a reaction while removing water to the outside of the system by azeotropic dehydration with water-insoluble solvent.

In addition, a combination of hydrophobicity of catalyst secondary amine and amount of water in the system is also important: When water is large in amount, a very hydrophobic catalyst should be used, but if an amount of water is small, a relatively less hydrophobic catalyst can be used.

This invention is further described in detail.

This invention concerns a process for the preparation of an α,β-unsaturated ketone by reacting an aldehyde with an alkaline metal salt of acetoacetic acid represented by the formula

$$CH_3 - \underset{\substack{\| \\ O}}{C}CH_2COO^-M^+$$

wherein $M^+$ is an alkaline metal ion, in the presence of a secondary amine, in a mixed solvent of water and a water-insoluble organic solvent, while keeping the pH constant with mineral acid, and by adjusting the amount of water, wherein as aldehyde 4-tetrahydropyran carbaldehyde, 2-tetrahydrofuran carbaldehyde, 3-tetrahydropyran carbaldehyde, 2-tetrahydropyran carbaldehyde or 3-tetrahydrothiopyran carbaldehyde and as amine 3,5-dimethylpiperidine is used.

The α, β-unsaturated ketones produced by the present process are α, β-unsaturated ketones having heterocyclic groups are 4-(3-tetrahydropyranyl)-3-butene-2-one, 4-(4-tetrahydropyranyl)-3-butene-2-one, 4-(2-tetrahydropyranyl)-3-butene-2-one 4-(2-tetrahydrofuranyl)-3-butene-2-one and 4-(3-tetrahydrothiopyranyl)-3-butene-2-one.

The aldehydes used in the present process are 4-tetrahydropyran carbaldehyde, 2-tetrahydrofuran carbaldehyde, 3-tetrahydropyran carbaldehyde, 2-tetrahydropyran carbaldehyde and 3-tetrahydrothiopyran carbaldehyde.

Methods to reduce the amount of water in the system are in addition to use of concentrated aqueous solution of alkaline metal salt of acetoacetic acid use of acid gas such as hydrogen chloride gas, or acid anhydride such as anhydrous sulfuric acid or phosphorus pentaoxide, or concentrated acid such as concentrated sulfuric acid or 85% phosphoric acid, as an acid to control the pH, or removal of water to the outside of the system by azeotropic dehydration with water-insoluble solvent during the reaction.

Water-insoluble organic solvents used in this reaction include chlorinated hydrocarbon solvents such as dichloromethane, chloroform and dichloroethane; and aromatic solvents such as benzene, toluene and xylene.

A way of implementing the synthetic method of this invention is described in detail:

To an aqueous solution of 1 to 3 moles of alkaline metal salt of acetoacetic acid to a mole of aldehyde was added 0.01 moles or more, preferebly 0.05 to 0.20 moles, to a mole of aldehyde, of catalyst secondary amine selected by taking into account the concentrations of alkaline metal salt of acetoacetic acid and of mineral acid used to adjust the pH, and further a mineral acid is added to adjust the pH to 6.0 to 8.0.

Then 10 to 500ml/mole (of aldehyde) of water-insoluble organic solvent is added together with aldehyde. If no solvent is used, β-hydroxyketone represented by general formula [IV] and described later is byproduced in a large amount, though the reaction proceeds. The reaction is carried out with stirring for 1 to 8 hours at 30 to 60 °C while keeping the pH to 6.0 to 8.0 with mineral acid. As the acid, in order not to increase the amount of water in the system, acids with less water content such as concentrated sulfuric acid and 85% phosphoric acid, or acid gas such as hydrogen chloride gas, or anhydrous acids such as anhydrous sulfuric acid and phosphorus pentaoxide are preferably used, if the aqueous solution of alkaline metal salt of acetoacetic acid is around 30% in concentration. If the aqueous solution of alkaline metal salt of acetoacetic acid is 50% or more in concentration, an acid with much water content such as concentrated hydrochloric acid may be used. It is possible to use an acid with much water content such as concentrated hydrochloric acid in a manner of removing water to the outside of the system during the reaction by azeotropic dehydration with water-insoluble solvent, if the concentration of alkaline metal salt of acetoacetic acid is around 30%. After the reaction is completed, water and water-insoluble organic solvent are added, the pH is adjusted to below 2 with mineral acid, and the organic layer is separated from the aqueous layer.

The organic layer is concentrated, and the obtained residue is distilled under reduced pressure to give the intended α, β-unsaturated ketone. β -hydroxyketones may be byproduced in less than 10%, depending on a combination of aldehyde, catalyst and an amount of water in the system. If so, after the reaction is completed, 0.10 to 2.00 moles, to aldehyde, of mineral acid such as sulfuric acid is added to the reaction mixture to heat, then β-hydroxyketones can be converted to the intended α, β-unsaturated ketone.

The aqueous layer separated from the organic layer is adjusted for the pH to 13 or more with hydroxide alkali such as sodium hydroxide and extracted with water-insoluble organic solvent to recover 90% or more of the catalyst amine used. The recovered can be used again.

Best Mode for carrying Out the Invention:

Implementation manner of this invention is further described in detail by reference to the following examples. The range of this invention is not limited at all by the following examples.

Example 1.

Into a reaction vessel of 500ml in inside volume were placed 116.0g 1.0 mole) of methyl acetoacetate and 102.5g of water, to which 144.3g (1.05 moles) of 29.1% NaOH aqueous solution was dropped over an hour with stirring while cooling with water and keeping the inside temperature to below 35 °C, and after it the resulting solution was continuously stirred at 33 to 37°C for 6 hours. Then water and methanol were distilled by aspirator at 40°C under reduced pressure. Part of the content in the flask was collected to titrate for pH with 1N-HCl standard aqueous solution. The obtained aqueous solution of sodium acetoacetate was 40% in concentration and the yield was 96.5% to methyl acetoacetate.

46.5g (0.15 moles) of 40% aqueous solution of sodium acetoacetate was placed in a reaction vessel of 100ml in inside volume, 1.13g (0.01moles) of 3,5-dimethylpiperidine was added, and the pH was adjusted to 7.0 with concentrated sulfuric acid. Into the resulting solution were added 10ml of chloroform and 11.4g of 4-tetrahydropyran carbaldehyde to react at 40 °C with stirring. The pH was maintained to 7.0 to 7.5 during the reaction by dropping concentrated sulfuric acid. After the reaction was completed, 10ml of water and 40ml of chloroform were added, the pH was adjusted to 1.5 with concentrated sulfuric acid, the organic layer was separated from the aqueous layer, and the solvent was distilled under reduced pressure. The remaining oily product was distilled under vacuum to give 15.0g of colorless oily product with boiling point of 91 to 95°C (0.1mmHg). (Crude yield: 97.2%) The obtained product was analyzed by gas chromatography to find that the intended product 4-(4-tetrahydropyranyl)-3-butene-2-one was 90.8% in purity. (Yield: 88.3%) 9.1% of a byproduct, 4-hydroxy-4-(4-tetrahydropyranyl)-butane-2-one was contained.

The same reaction was repeated. After the reaction was completed, 5.9g of concentrated sulfuric acid and 30ml of chloroform were added to the reaction mixture to heat to reflux at about 60°C for 2 hours. After cooled, 20ml of water was added, the organic layer was separated and washed with water, and the solvent was distilled under reduced pressure. The remaining oily product was distilled under vacuum to give 15.4g of colorless oily product . (Crude yield: 99.6%) The obtained product was analyzed by gas chromatography to find that the intended product 4-(4-tetrahydopyranyl)-3-butene-2-one was 96.8% in purity. (Yield: 96.4%) The product, if let stand at room temperature, crystallized. The crystal had melting point of 42 to 46 °C.

Examples 2-5

Example 1 was repeated using different aldehyde under the conditions shown in Table 1.

Example 6

The aqueous solution of sodium acetoacetate which was obtained by hydrolysis of aqueous sodium hydroxide solution of methylacetoacetate according to the method of Example 1 was 32% in concentration. 93.0g (0.24 moles) of the aqueous solution was placed in a reaction vessel of 200ml in inside volume, 2.26g (0.02 moles) of 3,5-dimethylpiperidine was added, and pH was adjusted to 7.0 with concentrated hydrochloric acid. Into the resulting solution were added 20ml of toluene and 26.0g (0.20 moles) of 3-tetrahydrothiopyran carbaldehyde, and a mixture of toluene and water was distilled by aspirator under reduced pressure at an inside temperature of 40°C. During the distillation, the pH was adjusted to 7.0 to 7.5 with concentrated hydrochloric acid and the same amount of toluene as that of toluene distilled was continuously added into the reaction vessel so that the amount of toluene in the vessel was always about 20ml. After 5 hours, the same post treatment as that used in Examaple 4 was carried out to give the

intended product of 4-(3-tetahydrothiopyranyl)-3-butene-2-one with yield of 88.2%.

Example 7

93.0g (0.24 moles) of 32% aqueous solution of sodium acetoacetate obtained in the same manner as that used in Example 35 was placed in a reaction veseel of 200ml in inside volume, 22.6g (0.02 moles) of 3,5-dimethylpiperidine was added, an pH was adjusted to 7.0 with concentrated hydrochloric acid. Into the resulting solution were added 20ml of toluene and 26.0g (0.20 moles) of 3-tetrahydrothiopyran carbaldehyde to react at 40°C for 5 hours with stirring. During the reaction, hydrogen chloride gas was blown into the reaction solution in order to keep the pH in the range of 7.0 to 7.5. After the reaction was completed, the solution was treated in the same manner as that used in Example 4. 4-(3-Tetrahydrothiopyranyl)-3-butene-2-one was obtained with yield of 89.5%. Boiling point: 107 - 108°C (0.12mmHg)

Example 8

93.0g (0.24 moles) of 32% aqueous solution of sodium acetoacetate obtained in the same manner as that used in Example 35 was placed in a reaction vessel of 200ml in inside volume, 2.26g (0.02 moles) of 3,5-dimethylpiperidine was added, and the pH was adjusted to 7.0 with concentrated sulfuric acid. Into the resulting solution were added 20ml of toluene and 13.0g (0.10 moles) of 3-tetrahydrothiopyran carbaldehyde to react at 40°C for 5 hours with stirring. During the reaction, liquid sulfur trioxide ion was dropped during the reaction solution in order to keep the pH in the range of 7.0 to 7.5. After the reaction was completed, the same post treatment as that used in Example 4 was carried out to give 4-(3-Tetrahydrothiopyranyl)-3-butene-2-one with yield of 88.9%. Boiling point: 106 - 108°C (0.10mmHg)

Table 1

| Compound No. | $R^1$ material $R^1CHO$ | $R^1$ product $R^1CH=CHCOCH_3$ | Catalytic amine mole ratio ( /aldehyde) | Concentration, mole ratio of aqueous solution of sodium acetoacetate ( /aldehyde) | solvent (ml/mole of aldehyde) | pH adjusting agent | Reaction temperature Reaction Time | yield No dehydration | yield with dehydration | Physical property value |
|---|---|---|---|---|---|---|---|---|---|---|
| 1 | 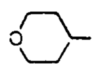 | | 3,5-dimethyl-piperidine 0,10 | 40 2.0 | chloroform 100 | C·$H_2SO_4$ | 40°C 5 hr | 88.3 | 96.4 | bp. 91 – 95 °C (0.1 mmHg) mp. 45–46°C |
| 2 | 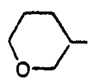 | | 〃 | 〃 | 〃 | 〃 | 〃 | 89.6 | 95.3 | bp. 102– 103 °C ( 2 mm Hg) $n_D^{26}$ 1.4783 |
| 3 | 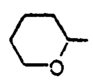 | | 〃 | 〃 | 〃 | 〃 | 40°C 4 hr | 83.4 | 92.4 | bp. 92 – 93 °C ( 5 mm Hg) $n_D^{25}$ 1.4709 |
| 4 | 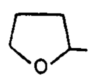 | | 〃 | 40 1.3 | 〃 | 〃 | 40°C 3 hr | 90.3 | 95.1 | bp. 78 – 81 °C ( 3 mm Hg) $n_D^{25}$ 1.4820 |

| Compound No. | $R^1$ material $R^1CHO$ | $R^1$ product $R^1CH=CHCOCH_3$ | Catalytic amine mole ratio ( /aldehyde) | Concentration, mole ratio of aqueous solution of sodium acetoacetate ( /aldehyde) | solvent (ml/mole of aldehyde) | pH adjusting agent | Reaction temperature Reaction Time | yield No dehydration | yield with dehydration | Physical property value |
|---|---|---|---|---|---|---|---|---|---|---|
| 5 | 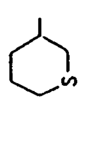 | | 3,5-dimethylpiperidine 0.10 | 40 1.2 | toluene 100 | C·$H_2SO_4$ | 40℃ 3hr | 89.8 | 92.2 | bp. 107 – 108℃ (0.1 mmHz) $n_D^{22}$ 1.5320 |
| 6 | 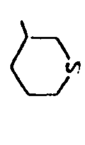 | | 3,5-dimethylpiperidine 0.1 | 32 1.2 | toluene 100 | C·HCl | 40℃ 5hr | 88.2 | 91.5 | |
| 7 | ″ | | ″ | ″ | ″ | HCl gas | ″ | 89.5 | | |
| 8 | ″ | | ″ | ″ | ″ | $SO_2$ | ″ | 88.9 | | |

[Effect of the Invention]

This invention is to provide methods for the synthesis of intended α, β-unsaturated ketones with heterocyclic groups with high yield under mild reaction conditions, by reacting an aqueous solution of

alkaline metal salt of acetoacetic acid with heterocyclic aldehydes in the presence of 3,5-dimethylpiperidine by adjusting an amount of water in the system. The invention is extremely significant in industry, particularly in manufacturing of agricultural chemicals and pharmaceuticals.

## Claims

**1.** A process for the preparation of an $\alpha,\beta$-unsaturated ketone by reacting an aldehyde with an alkaline metal salt of acetoacetic acid represented by the formula

$$CH_3 - \underset{\underset{O}{\|}}{C}CH_2COO^-M^+$$

wherein $M^+$ is an alkaline metal ion, in the presence of a secondary amine, in a mixed solvent of water and a water-insoluble organic solvent, while keeping the pH constant with mineral acid, and by adjusting the amount of water, characterized in that as aldehyde 4-tetrahydropyran carbaldehyde, 3-tetrahydropyran carbaldehyde, 2-tetrahydropyran carbaldehyde, 2-tetrahydrofuran carbaldehyde or 3-tetrahydrothiopyran carbaldehyde and as amine 3,5-dimethylpiperidine is used.

## Patentansprüche

**1.** Verfahren zur Herstellung eines $\alpha,\beta$-ungesättigten Ketons durch Umsetzung eines Aldehyds mit einem Alkalimetallsalz von Acetoessigsäure, angegeben durch die Formel

$$CH_3 - \underset{\underset{O}{\|}}{C}CH_2COO^-M^+$$

in der $M^+$ ein Alkalimetallion ist, in Gegenwart eines sekundären Amins, in einem gemischten Lösungsmittel aus Wasser und einem in Wasser unlöslichen organischen Lösungsmittel, während der pH-Wert mit Mineralsäure konstant gehalten wird, und durch Einstellung der Menge an Wasser, dadurch gekennzeichnet, daß als Aldehyd 4-Tetrahydropyran-carbaldehyd, 3-Tetrahydropyran-carbaldehyd, 2-Tetrahydropyran-carbaldehyd, 2-Tetrahydrofuran-carbaldehyd oder 3-Tetrahydrothiopyran-carbaldehyd und als Amin 3,5-Dimethylpiperidin verwendet wird.

## Revendications

**1.** Procédé pour la préparation d'une cétone $\alpha$-$\beta$-insaturée par réaction d'un aldéhyde avec un sel d'un métal alcalin d'acide acétoacétique représenté par la formule

$$CH_3 - \underset{\underset{O}{\|}}{C}CH_2COO^-M^+$$

où $M^+$ est un ion d'un métal alcalin, en présence d'une amine secondaire, dans un solvant mixte d'eau et d'un solvant organique insoluble dans l'eau, tout en maintenant le pH constant au moyen d'un acide minéral et en ajustant la quantité d'eau, caractérisé en ce que, comme aldéhyde, on utilise le 4-tétrahydropyrane carbaldéhyde, le 3-tétrahydropyrane carbaldéhyde, le 2-tétrahydropyrane carbaldéhyde, le 2-tétrahydrofuranne carbaldéhyde ou le 3-tétrahydrothiopyrane carbaldéhyde et comme amine, la 3,5-diméthylpipéridine.